# EUROPEAN PATENT SPECIFICATION

(11) **EP 0 975 802 B1**
(45) Date of publication and mention of the grant of the patent: **23.06.2004**
(21) Application number: 98913953.0
(22) Date of filing: 01.04.1998
(51) Int. Cl.: C12Q 1/68

(54) **METHOD OF NUCLEIC ACID SEQUENCING**
METHODE ZUR SEQUENZIERUNG VON NUKLEINSÄUREN
METHODE DE SEQUENCAGE D'ACIDE NUCLEIQUE

(30) Priority: 01.04.1997 GB 9706529; 23.06.1997 GB 9713236
(43) Date of publication of application: 02.02.2000
(73) Proprietor: Manteia S.A., 1267 Coinsins (CH)
(72) Inventor: KAWASHIMA, Eric, H., CH-1228 Plan Les Quates (CH); FARINELLI, Laurent, CH-1800 Vevey (CH); MAYER, Pascal, CH-1228 Plan Les Ouates (CH)
(74) Representative: De Luca, Giampiero
(86) International application number: PCT/GB1998/000963
(87) International publication number: WO 1998/044152

(56) References cited:
- EP-A- 0 665 293
- WO-A-91/06678
- WO-A-96/27025
- WO-A-96/32504
- WO-A-96/36737
- DE-A- 19 515 552
- FU D -J ET AL: "SEQUENCING DOUBLE-STRANDED DNA BY STRAND DISPLACEMENT" NUCLEIC ACIDS RESEARCH, vol. 25, no. 3, 1997, pages 677-679, XP000196977

## Description

The present invention relates to nucleic acid sequencing.

Nucleic acid sequencing methods have been known for many years. Two of the best known methods are the Sanger "dideoxy" method and the Maxam-Gilbert method.

The Sanger dideoxy method relies upon dideoxyribonucloside triphosphates being used as chain terminators. A DNA polymerase is used to extend a primer in order to synthesise a DNA sequence that is complementary to at least part the sequence of a target molecule. The primer extension reaction is performed in four different batches, each batch containing four radioactive deoxyribonucloside triphosphates and a dideoxy analogue of one of the deoxyribonucloside triphosphates. The dideoxy analogue is an analogue of a different deoxyribonucloside triphosphate in each batch. Once an analogue has been incorporated then no further primer extension can occur and the length of the strand formed is fixed. The strands of different length formed in each of the four batches are then size-fractionated in each of four different lanes of a gel (corresponding to the four different batches). This allows the sequence to be read from an autoradiograph.

In contrast to the Sanger dideoxy method, the Maxam-Gilbert method relies upon the chemical cleavage of labelled DNA. Here several identical DNA strands are each labelled at one end with a radioactive label. The strands are then split into four different groups. These groups are subjected to particular reaction conditions, which are different for each group. The reaction conditions are chosen to cause an average of about one chemical cleavage reaction per strand at a location where a particular base is present. The cleavage products of the four groups are then size fractionated on four different lanes of a gel (corresponding to cleavage at each of four different bases). By using autoradiography to read the gel the order in which the bases appear in the original DNA sequence can be determined.

Although the methods discussed above have been used extensively, they rely upon time-consuming sequencing from gels and are therefore not for the parallel sequencing of many different samples. Other methods, which do not require the use of gels, have therefore been designed for parallel sequencing.

WO 96/12039 (Lynx Therapeutics, Inc.) discloses a method for determining the nucleotide sequence of a target polynucleotide. The method comprising the steps of generating from the target polynucleotide a plurality of fragments that cover the target polynucleotide; attaching an oligonucleotide tag from a repertoire of tags to each fragment of the plurality such that substantially all the same fragments have the same oligonucleotide tag attached and substantially all different fragments have different oligonucleotide tags attached, the oligonucleotide tags being oligonucleotides of natural nucleotide monomers having a length in the range of from 10 to 20 nucleotides or oligonucleotides of natural nucleotide monomers comprising a plurality of subunits such that each subunit of the plurality consists of an oligonucleotide of natural nucleotide monomers having a length from three to six nucleotides, the subunits being selected from a minimally cross-hybridising set; sorting the fragments by specifically hybridising the oligonucleotide tags with their respective tag complements; determining the nucleotide sequence of a portion of each of the fragments of the plurality; and determining the nucleotide sequence of the target polynucleotide by collating the sequences of the fragments.

In one embodiment of the method disclosed in WO 96/12039, the step of determining the nucleotide sequence of the fragments is carried out simultaneously for a plurality of fragments by a single base sequencing method. A suitable single base sequencing method is said to be the method disclosed in international patent application PCT/US95/03678 (published as WO95/27080-Lynx Therapeutics, Inc.). This method comprises the steps of: ligating a probe to an end of a polynucleotide, the probe having a nuclease recognition site; identifying one or more nucleotides at the end of the polynucleotide; and cleaving the polynucleotide with a nuclease recognising the nuclease recognition site of the probe such that the polynucleotide is shortened by one or more nucleotides.

WO96/12014 (Lynx Therapeutics Inc.) also describes the molecular tagging system that is described in WO96/12039. However here various applications in addition to sequencing are discussed.

WO93/21340 (The Medical Research Council) discloses a sequencing method. This method comprises the steps of : forming a single-stranded template comprising the nucleic acid to be sequenced; hybridising a primer to the template to form a template/primer complex; extending the primer by the addition of a single labelled nucleotide; determining the type of the labelled nucleotide added onto the primer; removing or neutralising the label; repeating the preceding two steps sequentially and recording the order of incorporation of labelled nucleotides. The method is said to be suitable for use in an automated procedure whereby a large number of templates are sequenced simultaneously.

DE-A-4141178 (EMBL.) and WO96/27025 (Rabbani E.) disclose methods similare to those disclosed in WO93/21340. These are also said to be suitable for the simultaneous sequencing of several nucleic acids.

The method is for sequencing an at least partially single-stranded nucleic acid used as a template and uses a suitable polymerase together with four nucleotides in labelled form. Starting from a primer or a double-stranded nucleic acid section, a complementary strand is synthesised in a step-by step manner using the labelled nucleotides. The method includes the steps of incubating a nucleic acid which is provided with a primer or which is partially double-stranded with an incubation mixture consisting of a polymerase, one of the four labelled nucleotides and other substances needed for chain polymerisation so that a labelled nucleotide is incorporated into a growing complementary strand in the event that the next available nucleotide on the template-strand is complementary to the labelled nucleotide; separating the nucleic acid strand which may have been extended by one nucleotide in the previous step and carrying out one or more washing steps in known manner; determining whether a nucleotide has been incorporated by virtue of its label; and, if a nucleotide has successfully been incorporated, removing the label. The preceding steps are cyclically executed for all four nucleotides.

From the foregoing description it will be appreciated that several methods for parallel sequencing are known. However, although known methods for parallel sequencing have greatly increased the number of samples that can be sequenced in a given time, there is a need to provide an even greater throughput of samples. Many groups are attempting to sequence very large numbers of nucleic acid molecule samples and even complete genomes (as in the human genome sequencing project, for example). However they are limited by the efficiency of the sequencing techniques available. Furthermore most existing methods of parallel sequencing are costly, often requiring the use of large numbers of reagents and/or requiring many process steps. The presence of many different reagents and the use of many process steps is disadvantageous in that it can lead to sequencing errors.

The present invention aims to alleviate one or more of the disadvantages of known parallel sequencing methods.

According to the present invention there is provided a method for sequencing nucleic acid molecules, comprising the steps of:
a) providing at a first location of a surface a plurality of single stranded nucleic acid molecules that have the same sequences as one another and that are hybridised to primers in a manner to allow primer extension in the presence of nucleotides and a nucleic acid polymerase;
b) providing at a second location of a surface, which is different from the first location, a plurality of single stranded nucleic acid molecules that have the same sequences as one another, but that have different sequences from the sequences of the single stranded nucleic acid molecules at the first location, and that are also hybridised to primers in a manner to allow primer extension in the presence of nucleotides and a nucleic acid polymerase;
c) providing each location with nucleic acid polymerase and a given Labelled nucleotide under conditions that allow extension of the primers if a complementary base or if a plurality of such bases is present at the appropriate position in the single stranded nucleic acid molecules;
d) detecting whether or not said labelled nucleotide has been used for primer extention at each location by determining whether or not the label present on said nucleotide has been incorporated into extended primers;
e) repeating steps c) and d) one or more times so that extended primers comprising a plurality of labels are provided.

By providing different template molecules at different locations, the present invention allows the sequencing of different nucleic acid molecules to occur in parallel.

The present invention allows labelled nucleotides to be incorporated in a stepwise manner in a nucleic acid molecule via primer extension. However the presence of one labelled nucleotide in the molecule does not prevent other labelled nucleotides being detected (even if adjacent labelled nucleotides are the same). Thus, unlike many prior art parallel sequencing methods, such as those disclosed in WO93/21340 and DE-A-4141178, there is no need to remove a label from a polynucleotide chain before a further labelled nucleotide is added (although in some embodiments it may be desired to remove labels periodically). A plurality of labels can therefore be incorporated into a nucleic acid molecule via primer extension and can be detected *in situ* by measuring the cumulative increase of the signal generated by the label on said nucleotides.

Furthermore, as discussed in greater detail later on, in one embodiment the present invention is particularly advantageous in sequencing nucleic acid strands comprising one or more stretches of contiguous identical bases (e.g. polyA, polyT, polyC, polyU or polyG stretches). This is because the number of steps required for sequencing such strands can be significantly reduced.

The present invention is also advantageous over prior art methods that rely upon the use of large numbers of different oligonucleotide tags in determining nucleotide sequences, such as the methods disclosed in WO96/12014 and WO96/12039. These methods are generally time-consuming and require specific tags to be provided. They may also use restriction enzyme digestion or other means to remove tags or parts thereof following a detection step. The present invention does not suffer from these drawbacks.

A further advantage of the present invention over various prior art methods is that it allows high sensitivity because labels can be directly incorporated into a plurality of identical nucleic acid molecules that are present at a single location. As described later, the incorporation of labelled nucleotides (e.g. fluorescence-labelled nucleotides) and detection by a sensitive cooled CCD camera can allow a sharp signal to be produced and detected *in situ.* The CCD camera is preferably mounted on an inverted microscope.

The single stranded molecules that are hybridised to primers in steps a) and b) of the method of the present invention are referred to herein as "templates". These may be DNA or RNA molecules. They may comprise naturally occurring and / or non-naturally occurring nucleotides.

Preferably the templates are immobilised on a surface - i.e. they are maintained in position on a surface by covalent or non-covalent interactions for sufficient time to allow the present invention to be performed. They may be directly or indirectly bound to the surface.

Sequencing may be full or may be partial. The sequence of a complementary strand to the template or of a part thereof can be obtained initially. However this sequence can be converted (using base-pairing rules) to provide the sequence of the template or of a part thereof. This conversion can be done via a computer or via a person. It can be done after each step of primer extension or at a later stage.

Desirably, the method of the present invention includes a step of removing labelled nucleotides from the first and second locations if they are not incorporated by primer extension (e.g. via a washing step, as will be described later). By immobilising templates, unincorporated labelled nucleotides can be removed from the locations of the templates whilst the templates are maintained in position.

Any suitable surface may be provided for immobilising nucleic acid molecules. The term "support" is used herein to describe a material possessing such a surface. The support is preferably a solid material or a semi-solid material (e.g. a gel or a lipidic monolayer / bilayer).

Various protocols are known for binding nucleic acids to a surface. For example they can be covalently bound to the surface of a glass or polymer support (see e.g., Joos, B., Kuster, H., and Cone, R. (1997), entitled "Covalent attachment of hybridizable oligonucleotides to glass supports", *Analytical Biochemistry* **247**, 96-101; Oroskar AA, Rasmussen SE, Rasmussen HN, Rasmussen SR, Sullivan BM, Johansson A. (1996), entitled "Detection of immobilized amplicons by Elisa-like techniques", Clinical Chemistry. 42(9):1547-1555; Khandjian EW, entitled "UV crosslinking of RNA to nylon membrane enhances hybridization signals", Molecular Biology Reports. 11(2):107-15, 1986.)

Nucleic acid molecules may also be immobilised to a support via non-covalent binding. The non-covalent binding may be achieved, for example, by interaction between biotinylated-nucleic acids and a (strept)avidin coated surface or by the anchoring of a hydrophobic chain into a lipidic monolayer or bilayer.

Indeed any interaction between a surface and a nucleic acid molecule which allows the various steps of the method to be performed may be utilised for binding. For example template molecules may be bound to a surface merely by base pairing with primers already bound to a surface.

In preferred embodiments, a single surface is provided on which template molecules are immobilised. In less preferred embodiments, discrete units each having their own surface may be provided. For example, spheroids may be provided (e.g. glass beads/beads formed of another polymeric material, etc.). Alternatively other three-dimensional units may be provided.

The discrete units may possess magnetic properties or other properties to facilitate separation of the units from other components, e.g. during washing steps. (Magnetic beads can be obtained from Dynabeads™ M-280, Dynal A.S. Oslo, Norway. They can be used as described by Hultman, T., Bergh, S., Moks, T., Uhlen, M. Bidirectional solid-phase sequencing of *in vitro*-amplified plasmid DNA. BioTechniques 10:84-93, 1991.)

Different units may even have different properties in order to facilitate separation into different categories of unit (e.g. primers of one type may be bound to units having a given property and primers of another type may be bound to units having a different property or at least not having the given property).

Desirably, a planar surface is provided (e.g. by a support which is- a membrane or is a glass slide).

It is preferred to provide high densities of immobilised template molecules and/or of primers. Desirably high densities are provided in small areas. This can be achieved using robotic systems. Such systems may rely on piezoelectric delivery systems or other delivery systems in order to deliver small quantities (e.g. nanolitres or even picolitres) of material to small areas. The small areas may be in the form of arrays (that may be regular or irregular). By controlling the operation of a delivery device, different types of array may be provided.

In less preferred embodiments arraying can be performed after binding of template and/or primer molecules to a surface. In such a case the molecules can be immobilised on discrete units of support (*e.g.* as discussed above), which are subsequently arrayed. Each unit may therefore have a surface on which immobilised nucleic acids are present.

Desirably, a large number of copies of the same initial template molecule is provided at a given location. For example, over 10,000 such molecules may be provided.

A location may be in the form of a single distinct area (that is preferably planar). Preferably the areas are from 100 nm to 2 cm, more preferably from 500 nm to 5 mm in length, when measured across the largest dimension. In the case of areas defined by a generally circular perimeter this measurement will be the diameter of the circle.

Less preferably, a location may comprise the contents of a container or a part thereof (e.g. template-bound beads in a tube or in a microplate well).

A plurality (two or more) of locations having one or more of the characteristics discussed in the foregoing paragraphs will be generally provided.

Where a large number of template molecules are provided at the locations, this can enhance the reliability of sequence data provided. This is because the probability of a false reading being provided due to the presence of contaminants can be minimised.

In preferred embodiments, the sequencing may be performed on a small number of template molecules provided that sensitive detection techniques are used.

Sensitive detection techniques allowing for detection of fluorescently-labelled molecules can, for example, utilise:
a) A cooled Charge Coupled Device (CCD) camera (e.g. Princeton Instruments),
b) Confocal microscopy (e.g. Carl Zeiss Jena),
   or
c) High-speed DNA sequencing: an approach based upon fluorescence detection of single molecules. (Journal of Biomolecular Structure & Dynamics 7: 301-309, Jett, J.H., Keller, R.A., Martin, J.C., Marrone, B.L., Moyzis, R.K., Ratliff, R.L., Seitzinger, N.K., Brooks Shera, E., Stewart, C.C. (1989)).

Template molecules provided at different locations may be nucleic acid molecules from different sources. These may or may not have one or more identical/homologous sequences along their lengths. For example, different template molecules may have identical/homologous sequences over all/part of their lengths when similar samples from different organisms are provided (e.g. similar genetic regions from related organisms). Alternatively, different samples from the same organism (e.g. samples from different types of cell, tissue or organ) may be provided.

One of the main advantages of the present invention is that sequences, whether full or partial, can be read simultaneously and efficiently from a plurality of different locations. (The present invention is not limited to being applied at only the first and second locations discussed previously.) For example, over 10, over 100, over 1000, or even over 1,000,000 different locations may be provided. Thus many different sequences can be determined in a relatively short time period.

If desired, each type of template molecule can be provided at a plurality of different locations (preferably in amplified form at each location), allowing for redundancy of the sequence data generated. This redundancy allows controls to be provided to provide checks on the accuracy of sequence data generated.

Oligonucleotides are preferred as primers suitable for use in the present invention. These are nucleic acid molecules that are typically 6 to 60, e.g. 15 to 25 nucleotides long. They may comprise naturally and / or non-naturally occurring nucleotides. Other molecules, e.g. longer nucleic acid strands may alternatively be used as primers, if desired.

Primers can be annealed (hybridised) to template molecules. Primers which remain in solution or which do not anneal specifically to the template are preferably removed after annealing. Preferred annealing conditions (temperature and buffer composition) prevent non-specific hybridisation. These may be stringent conditions. Such conditions would typically be annealing temperatures close to a primer's Tm (melting temperature) at a given salt concentration (e.g. 50 nM primer in 200 mM NaCl buffer at 55°C for a 20-mer oligonucleotide with 50% GC content).

(Stringent conditions for a given system can be determined by a skilled person. They will depend on the base composition, GC content, the length of the primer used and the salt concentration. For a 20 base oligonucleotide of 50% GC, calculated average annealing temperature is 55-60°C, but in practice may vary between 35 to 70°C).

Primers may be immobilised by being directly linked to a surface by the techniques discussed *supra* for template molecules. Alternatively, they may be indirectly linked to the surface, e.g. by virtue of being annealed to a template molecule that is itself bound to the surface.

In any event the template molecule will comprise a portion that hybridises with the primer (preferably under "stringent" conditions). This portion can be added to a given molecule (even if of a totally/partially unknown sequence) using techniques known to those skilled in the art to provide a template. For example it can be synthesised artificially and can be linked via a ligase to the molecule of totally/partially unknown sequence. This can result in a single or double stranded molecule. If a double stranded molecule is produced, heating can be used to separate annealed strands to provide a single stranded template, which can then be annealed to a primer.

In addition to the ways of providing a template annealed to a primer discussed above, a template annealed to a primer can also be provided by starting with a double stranded molecule and then removing part of one of the strands. This will leave a part of one strand (the primer) annealed to a longer strand. Here the longer strand will comprise a single stranded portion to be sequenced.

The step of removing part of one of the strands can be achieved for example by using one or more nucleases. One way of doing this is by using limited digestion with a 3' → 5' exonuclease to leave a short strand with a free 3' OH group (the primer) hybridised to a larger strand (the template). (The longer strand can be capped at its 3' end to prevent it being digested by the exonuclease.) Another way is to introduce a nick into one strand with an endonuclease and then to remove part of that strand with a 5'→3' exonuclease. This will leave a free 3' OH group on a short strand (the primer) hybridised to a longer strand (the template). (The longer strand can be capped at its 5' end to prevent it being digested by the exonuclease.)

By whatever means the primer annealed to the template is provided, as discussed previously, it is preferred that the primer or the template molecule is immobilised. Immobilisation can be performed either before or after providing the primer annealed to the template.

Once a template annealed to a primer is provided, primer extension can be performed. RNA or DNA polymerases can be used. DNA polymerases are however the enzymes of choice for preferred embodiments. Several of these are commercially available. Polymerases which lack 3' → 5' exonuclease activity can be used, such as T7 DNA polymerase or the small (Klenow) fragment of DNA polymerase I may be used [*e.g.* the modified T7 DNA polymerase Sequenase™ 2.0 (Amersham) or Klenow fragment (3'→5' exo-, New England Biolabs)]. However it is not essential to use such polymerases. Indeed, where it is desired that the polymerases have proof-reading activity polymerases lacking 3' → 5' exonuclease activity would not be used.

Certain applications may require the use of thermostable polymerases such as ThermoSequenase™ (Amersham) or Taquenase™ (ScienTech, St Louis, MO).

Any nucleotides may be used for primer extension reactions (whether naturally occurring or non-naturally occurring). Preferred nucleotides are deoxyribonucleotides; dATP, dTTP, dGTP and dCTP (although for some applications the dTTP analogue dUTP is preferred) or ribonucleotides ATP, UTP, GTP and CTP; at least some of which are provided in labelled form.

The use of labelled nucleotides during primer extension facilitates detection. (The term "label" is used in its broad sense to indicate any moiety that can be identified using an appropriate detection system. Preferably the label is not present in naturally occurring nucleotides.)

Ideally, labels are non-radioactive, such as fluorophores which allow efficient detection of primer extension.

In some applications, considering that a large number of copies of each template molecules can be immobilised at each location, one can envisage the use of a combination of labelled and non-labelled nucleotides. In this case, even if a small proportion of the incorporated nucleotides are labelled (e.g. fluorescence-labelled), the number of labels at each location can be sufficient to be detected by a detection device. For example the ratio of labelled:non labelled nucleotides may be chosen so that labelled nucleotides are used in primer extension less than 50%, less than 20% or less than 10% of the time.

Thus in one embodiment of the present invention there is provided a method for sequencing nucleic acid molecules, comprising the steps of:
a) providing at a first location a plurality of single stranded nucleic acid molecules that have the same sequences as one another and that are hybridised to primers in a manner to allow primer extension in the presence of nucleotides and a nucleic acid polymerase;
b) providing at a second location, which is different from the first location, a plurality of single stranded nucleic acid molecules that have the same sequences as one another, but that have different sequences from the sequences of the single stranded nucleic acid molecules at the first location, and that are also hybridised to primers in a manner to allow primer extension in the presence of nucleotides and a nucleic acid polymerase;
c) providing each location with a nucleic acid polymerase and a given nucleotide in labelled and unlabelled form under conditions that allow extension of the primers if a complementary base or if a plurality of such bases is present at the appropriate position in the single stranded nucleic acid molecules;
d) detecting whether or not said labelled nucleotide has been used for primer extension at each location by determining whether or not the label present on said nucleotide has been incorporated into extended primers;
e) repeating steps c) and d) one or more times.

This embodiment of the present invention can reduce costs, since few labelled nucleotides are needed. It can also be used to reduce quenching effects, should this be a problem if certain labels are used where the signals from such labels interfere with each other.

The aspects of the other embodiments of the present invention discussed herein apply *mutatis mutandis* to the foregoing embodiment of the present invention.

In other embodiments of the present invention all or most of the nucleotides used can be labelled and primer extension can therefore result in the contiguous incorporation of labelled bases.

In one of these other embodiments, only one type of label is present on four types of nucleotides to be used in extending a primer. Each nucleotide incorporation can therefore provide a cumulative increase of the same signal (e.g. of a signal measured at a particular wavelength).

After several steps of sequencing during which a plurality of labelled nucleotides have been incorporated into a nucleic acid strand by primer extension, it may be desired to reduce a signal to an initial level or at least to some extent. This can be achieved by removing one or more labels from already incorporated nucleotides (*e.g.* by laser bleaching of the fluorophores).

Alternatively, polymerisation steps may proceed with another type of label from that used initially (*e.g.* switching from fluorescein to rhodamine).

In less preferred embodiments, different labels may be used for each type of nucleotide. For example, a different fluorophore for each dNTP may be used.

In other less preferred embodiments, the primer itself and its extension product may be removed and replaced with another primer. If required, several steps of sequential label-free nucleotide additions may be performed before actual sequencing in the presence of labelled nucleotides is resumed.

A washing step is preferably incorporated after each primer extension step in order to remove unincorporated nucleotides which may interfere with subsequent steps. The preferred washing solution should be compatible with polymerase activity and have a salt concentration which is high enough not to interfere with the annealing of primer molecules and templates.

In less preferred embodiments, the washing solution may interfere with polymerase activity. Here the washing solution would need to be removed before further polymerisation occurred.

Various detection systems can be used to detect labels (although in certain embodiments detection may be possible simply by eye, so that no detection system is needed). A preferred detection system for fluorescent labels is a Charge-Coupled-Device (CCD) camera, possibly coupled to a magnifying device. Any other device allowing detection and, preferably, also quantification of fluorescence on a surface may be used. Devices such as fluorescent imagers or confocal microscopes may be chosen.

In less preferred embodiments, the labels may be radioactive and a radioactivity detection device would then be required. Ideally such devices would be real-time radioactivity imaging systems. Less preferred are other devices relying on phosphor screens (Moleculal Dynamics) or autoradiography films for detection.

In embodiments of the method where template molecules are immobilised in containers (e.g. wells), sequencing reactions can be monitored for each container. This can be done using microplate readers to achieve high orders of parallelism. Such readers are available commercially for the measuring of fluorescence or radioactivity (e.g. Discovery™, FluoroCount™ or TopCount™ microplate readers from Packard Instrument Company).

Depending on the number of locations where detection of signals is desired, a scanning system may be preferred for data collection. (Although an alternative is to provide a plurality of detectors to enable all locations to be covered.) Such a system allows a detector to move relative to a plurality of locations to be analysed. This is useful when all the locations providing signals are not within the field of view of a detector. The detector may be maintained in a fixed position and locations to be analysed may be moved into the field of view of the detector (e.g. by means of a movable platform). Alternatively the locations may be maintained in fixed position and the detection device may be moved to bring them into its field of view.

The detection system is preferably used in combination with an analysis system in order to determine the number (and preferably also the nature) of bases incorporated by primer extension at each location after each step. This analysis may be performed immediately after each step or later on, using recorded data. The sequence of template molecules immobilised at a given location can then be deduced from the number and type of nucleotides added after each step.

Preferably the detection system is part of an apparatus comprising other components. The present invention includes an apparatus comprising a plurality of labelled nucleotides, a nucleic acid polymerase and detection means for detecting labelled nucleotides when incorporated into a nucleic acid molecule by primer extension, the detection means being adapted to distinguish between signals provided by labelled nucleotides incorporated at different locations.

The apparatus may also include temperature control, solvent delivery and washing means. It may be automated.

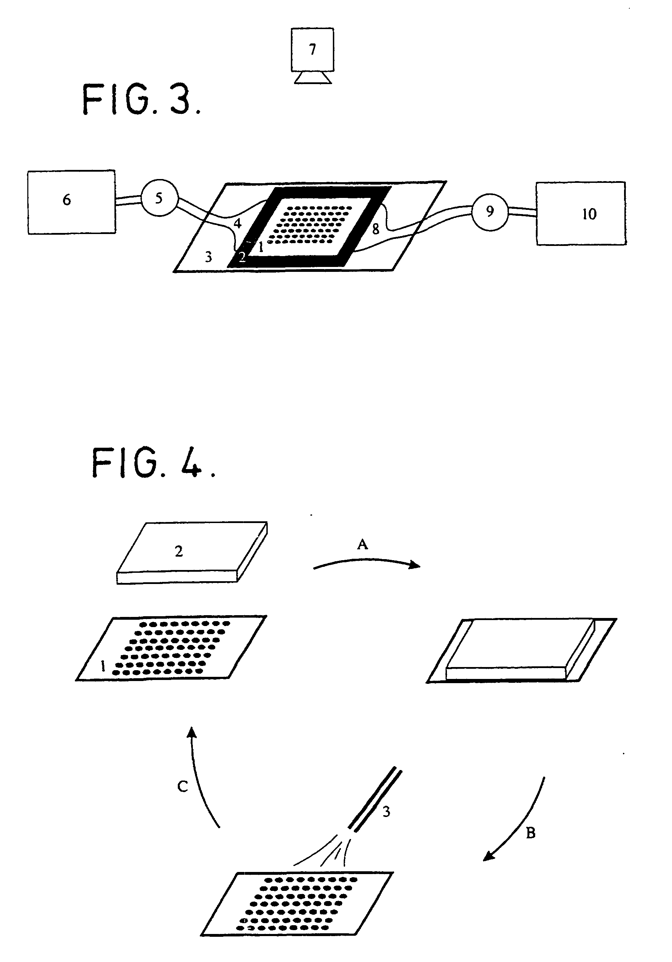

The elements forming such apparatus can have various shapes and can be arranged in different ways, as described in the examples (see in particular figures 3, 5, and 6). Thus, the skilled person in the art can appreciate the features and the advantages of each type of apparatus for performing the methods of the present invention in a much more precise and effective way when compared to the apparatus generically disclosed in the prior art (WO 96/27025).

Preferably, an apparatus for performing the methods of the invention comprises:
a) nucleic acids arrayed in the form of distinct areas, wherein a plurality of single stranded nucleic acid molecules that have the same sequences as one another, and that are hybridised to primers in a manner to allow primer extension in the presence of nucleotides and a nucleic acid polymerase, are arrayed in each of said distinct areas;
b) means for dispensing the sequencing reagents;
c) means for removing the sequencing regents;
d) automated means to allow repeated cycles of primer extension;
e) detection means being adapted to distinguish between said different locations whether or not the label present on the nucleotides has been incorporated into extended primers.

More preferably, the apparatus comprises nucleic acids that are arrayed in distinct areas on a planar surface. The means for dispensing and removing the sequencing reagents are formed, respectively, by one or more inlets and outlets for reagents connected to electronically controlled valves and reservoirs for reagents. Automated means, which are formed by the electronically controlled valves for dispensing and removing the reagents, allow repeated cycles of primer extension and detection. The detection means for distinguishing amongst the distinct areas whether or not the label present on the nucleotides has been incorporated into extended primers, are formed by a Charge-Coupled-Device camera coupled to a magnifying device.

An apparatus as described above may be used in a highly preferred embodiment of the present invention. Here different template molecules can be sequenced as follows:
1. each type of DNA molecule is arrayed and covalently bound at a different location (e.g. a spot) of a flat solid surface;
2. the molecules are denatured to produce single-stranded DNA;
3. oligonucleotide primers are specifically annealed to the single-stranded DNA template molecules; unbound primers are removed;
4. a reagent mixture for nucleotide extension (containing e.g. a single type of deoxyribonucleotide triphosphate, at least part of which are fluorescently-labelled, a DNA polymerase and a suitable reaction buffer) is applied to the DNA array;
5. the DNA array is washed to remove unincorporated nucleotides;
6. the amount of fluorescence at each location is measured and recorded; the reaction proceeds to step 4) using another type of nucleotide until a sufficient amount of sequence data has been generated.

Methods and apparatuses within the scope of the present invention can be used in the sequencing of:
- unidentified template molecules (i.e. *de novo* sequencing);
- and templates which are to be sequenced to check if one or more differences relative to a known sequence are present (e.g. identification of polymorphisms). This is sometimes referred to as "re-sequencing".

For *de novo* sequencing applications, the order of nucleotides applied to a given location can be chosen as desired. For example one may choose the sequential addition of nucleotides dATP, dTTP, dGTP, dCTP; dATP, dTTP, dGTP, dCTP; and so on. (Generally a single order of four nucleotides would be repeated, although this is not essential.)

The number of steps required for *de novo* sequencing a sequence of *n* bases is dependent on the template sequence: a high number of identical bases repeated in a contiguous stretch reduces the number of steps, whereas a template sequence having fewer such tandem repeats increases the number of steps. Without any identical base repeated contiguously, the maximum number of *de novo* sequencing steps required is 3*n*+ 1. (After a given base has been incorporated, the next based to be added is one of the 3 other bases. The exception to this is the first step of primer extension where the first base is any of 4 bases.) Thus, for templates without any repeated bases, the probable average number of steps required to sequence n bases is very close to 2*n* (considering that any of the 3 bases which may get incorporated at a given cycle has an equal probability to be added, thus meaning that in average a base will be added every 2 cycles). Thus, for biological templates which generally contain numerous bases in tandem, the actual number of cycles required for *de novo* sequencing will be lower than 2 times the number of bases to be sequenced. For a truly random sequence, it can be shown that the number of steps required to sequence *n* bases is, in average, 1.5*n* steps.

For re-sequencing applications, the order of nucleotides to be added at each step is preferably chosen according to a known sequence.

Re-sequencing may be of particular interest for the analysis of a large number of similar template molecules in order to detect and identify sequence differences (*e.g.* for the analysis of recombinant plasmids in candidate clones after site directed mutagenesis or more importantly, for polymorphism screening in a population). Differences from a given sequence can be detected by the lack of incorporation of one or more nucleotides present in the given sequence at particular stages of primer extension. In contrast to most commonly used techniques, the present method allows for detection of any type of mutation such as point mutations, insertions or deletions. Furthermore, not only known existing mutations, but also previously unidentified mutations can be characterised by the provision of sequence information.

In some embodiments of the method, long template molecules may have to be re-sequenced by several sequencing reactions, each one allowing for determination of part of the complete sequence. These reactions may be carried out at different locations (e.g. each location with the same template but with a different primer), or in successive cycles (e.g. between each cycles the primers and extension products are washed off and replaced by a different primer).

The present invention will now be described by way of example only, with reference to the accompanying drawings. Unless the context indicates otherwise, techniques described with regard to DNA molecules should also be considered to be applicable to RNA molecules.
FIGURE 1 illustrates in schematic form the *in situ* sequencing of two different DNA molecules, each molecule being present in multiple copies at particular locations, as indicated by squares or circles.
FIGURE 2 illustrates how fluorescence readings can be used to determine a DNA sequence *in situ.*
FIGURE 3, 4, 5 and 6 illustrate apparatuses of the present invention.
FIGURES 7, 8 and 9 illustrate that *in situ* sequencing done via the present invention can be confirmed (if desired) by standard techniques using gels.
FIGURES 10 and 11 illustrate *in situ* sequencing using fluorescent labels.

### Figure 1

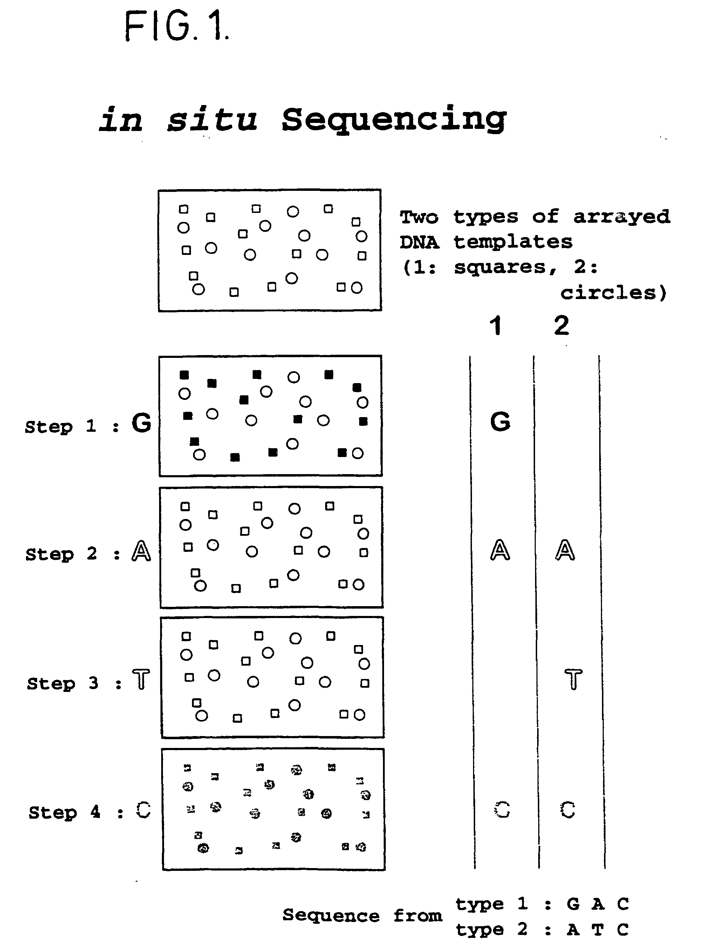

Referring now to Figure 1, individual spots or arrayed DNA templates are provided at different locations, each comprising large numbers of identical nucleic acid molecules.

Figure 1(a) shows the DNA templates before fluorescence labelled nucleotides are added. In this example, two different DNA templates are represented: template 1 by squares and template 2 by circles.

Figure 1(b) shows the DNA templates after a fluorescently-labelled dGTP has been added in the presence of a DNA polymerase and the DNA templates have been washed to remove any labelled dGTP not used in primer extension. The DNA templates which have incorporated one or more labelled Gs are represented by the black-filled squares.

Figures 1(c), (d) and (e) show how the procedure illustrated by Figure 1(b) has been repeated using labelled nucleotides dATP, dTTP and dCTP respectively (and washing after each step to remove nucleotides not used in primer extension).

Fluorescence detection can be used to distinguish incorporation of fluorescence at each location. The latest base to be incorporated at a given location by a primer extension is illustrated in Figures 1 (b) to I (e) by different appearances for different bases (G is represented by black colour, A by oblique shading, T by white colour and C by dots).

Figure 1(f) shows two partial sequences which have been determined by the method illustrated in Figures 1(a) to (e). The sequence shown in lane 1 is GAC, whereas that shown in lane 2 is ATC. For ease of reference, the DNA templates shown in Figure 1(a) have been identified with numbering corresponding to the lane numbering shown in Figure 1(f). It can thus be seen that two different types of DNA template are present in Figure 1(a) (i.e. a given DNA molecule is present in DNA templates identified with "1" in substantially homogenous form and with a different DNA molecule is present in DNA templates identified with "2", also in substantially homogenous form).

Of course the present invention can be used to sequence many more than two different nucleic acid molecule sequences and can be used for sequencing RNA as well as DNA.

### Figure 2

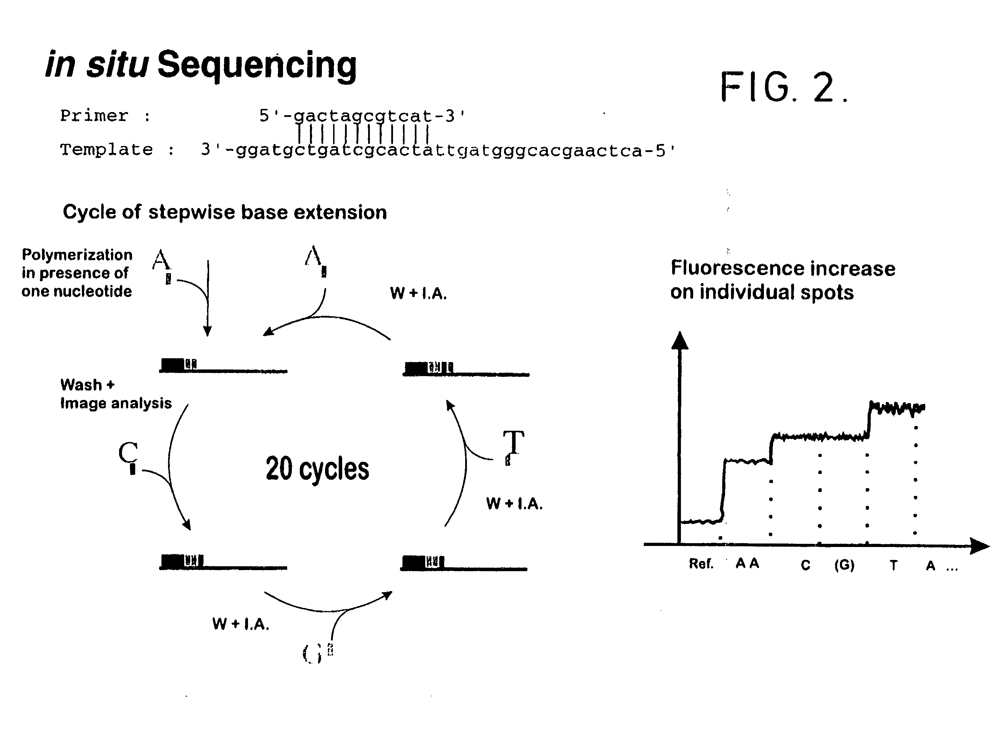

Turning now to Figure 2, *in situ* sequencing is illustrated using a particular primer annealed to a template.

Figure 2(a) shows the primer annealed to the template. The primer can be seen to have a 3' end available for primer extension.

Figure 2(b) shows how fluorescence-labelled bases complementary to the underlined bases shown in Figure 2(a) can be incorporated by a stepwise primer extension cycle using fluorescently-labelled nucleotides.

Figure 2(c) shows how bases with fluorescent labels, which can be incorporated by primer extension, can be detected *in situ* by fluorescence measurements.
The graphic illustrates fluorescence intensity values at a given location.

### Figure 3

Turning now to Figure 3, an apparatus is illustrated for performing the present invention.

The features shown are listed below:
1. Arrayed DNA samples and transparent cover plate.
2. Seal around cover plate.
3. Bottom plate.
4. Inlet for reagents. (A plurality of conduits could be provided.)
5. Electronically controlled valves.
6. Reservoir of reagents.
7. Detection device (e.g. CCD camera).
8. Outlet for reagents.
9. Electronically controlled valves.
10. Reservoir of reagents. N.B. The reagents can be recycled directly into reservoir 6.

In use, template molecules are arrayed onto a plate and are covered by a transparent cover (*e.g.* a quartz plate and a cover slip may be provided). The sides of the plate and cover are sealed so that liquid can be circulated between them. The reagents, which can be recycled, are distributed via a system of conduits and valves controlled electronically. Ideally a computer is used to program the sequential incubation of the samples with appropriate reagents under appropriate conditions. The same or another computer may also control the detection system (*e.g.* a CCD camera and possibly means for moving the camera relative to the template molecules) and perform data analysis.

### Figure 4

Figure 4 illustrates another apparatus for performing the present invention. This apparatus is preferred for manually controlled stepwise sequencing. The features illustrated are listed below:
1. Arrayed DNA samples.
2. Deformable absorbent material containing reagents, including appropriate type of nucleotide, to be used in primer extension.
3. Washing device.

In use, the deformable absorbent material (*e.g.* agarose gel or cellulose fibres) holds sequencing reagents (*e.g.* polymerase, buffer, and one type of nucleotide at a time). A soft pressure of the deformable absorbent material onto the surface where the template molecules are arrayed can liberate enough reagents for a primer extension reaction to occur, with minimal reagent waste. A washing step may then be performed by causing liquid to flow over the arrayed DNA samples, using the washing device.

### Figure 5

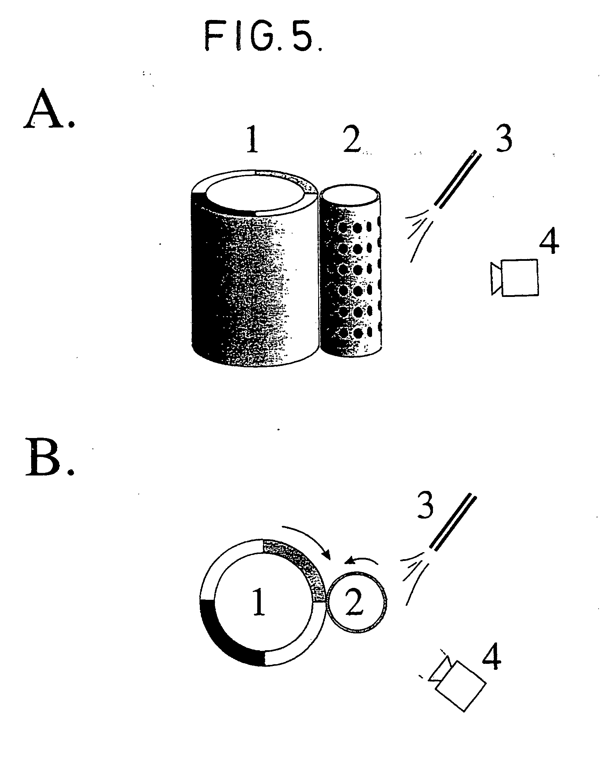

An automated system, as shown in Figure 5, can also be designed which uses the deformable absorbent material and reagents held thereon. Ideally, four types of reagent mixes (one for each type of nucleotide) are provided by separate areas of the deformable absorbent material. The deformable absorbent material may be generally cylindrical in shape and may have four different regions provided on the outer surface of a cylinder. The template molecules may be arrayed on the surface of another cylinder. The template cylinder may be of smaller diameter *(e.g. dt = 1*/*4dr* where *dt* is the diameter of the template cylinder and *dr* is the diameter of the reagents cylinder) or larger diameter (e.g. *dt* = *dr* + 1/4 or *dt* = *dr* + 3/4). A continuous cycling of reactions can be achieved by rolling the reagent and sample cylinders against each other.
Washing and detection can also be performed continuously along the surface of the template cylinder.

The automated system illustrated by Figure 5 will now be described in greater detail:

(Figure 5A shows a perspective view of the system. Figure 5B shows a plan view of the system.)

The components are as follows:
1.: Cylinder covered with deformable absorbent material containing sequencing reagents. Shown here is an example of cylinder with 4 sectors, which may each contain a different reagent (e.g. polymerisation mixtures, each one with a different nucleotide).
2.: Cylinder of smaller diameter (e.g. one fourth of the diameter of cylinder 1, as shown here) on which the samples are arrayed.
3.: Washing device.
4.: Detection device (e.g. CCD camera).

Typically, both cylinders roll against each other, so that the reagents on cylinder 1 are applied on surface of cylinder 2. The order of reagents application would correspond to their disposition on cylinder 1.

It is possible to modify this order of reagent application in other embodiments of the invention (e.g. for re-sequencing). For example, an additional device can be used to skip application of one or more reagents onto cylinder 2. This can be achieved by controlled rotation of cylinder 1 relative to cylinder 2 while contact between both cylinders is avoided.

### Figure 6

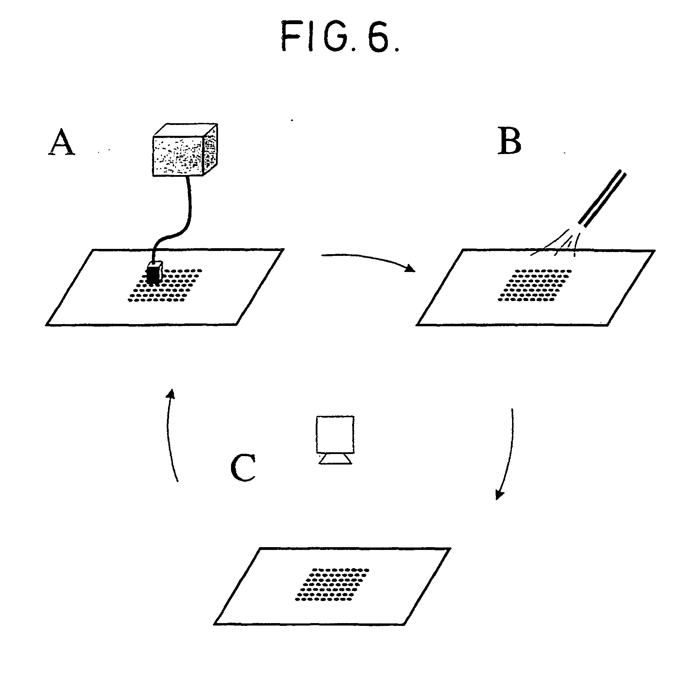

A further apparatus, as shown in Figure 6, can be designed. Reagents can be dispensed by microspraying. Thus minimal volumes of reagents need be used at each step. For instance, automated piezoelectric devices can be designed to dispense nanolitres (or picolitres) of reagents on defined locations (e.g. ink-jet printing technology). The re-sequencing of different templates, or of similar templates from different primers, can be achieved by dispensing the appropriate reagents (e.g. primer in annealing solution or polymerase, buffer and nucleotide) at defined locations.

Three systems that can be used successively to carry out a sequencing reaction are illustrated in Figure 6:
A. Piezoelectric device for dispensing the appropriate reagents at defined locations. The device is linked to an electronic controller and to a reservoir of reagents.
B. Washing device.
C. Detection device.

### Figure 7

Illustration of the sequencing method with 32P-labelled primer, as described in Example 1. The template was immobilised on magnetic beads. The sequencing cycles were carried out using the indicated nucleotides in an arbitrary order (A, T, G, C,..) to mimic *de novo* sequencing. The reaction products were visualised by autoradiography after electrophoresis on denaturing acrylamide gel. Fragment sizes are indicated on the right with the predicted sequence.

### Figure 8

Illustration of the sequencing method with 32P-labelled nucleotides, as described in Example 2. The template was immobilised on magnetic beads. The sequencing steps were carried out using the indicated nucleotides in the order of expected incorporation. The primer was unlabelled. The reaction products were visualised by auto-radiography after electrophoresis on denaturing acrylamide gel. Fragment sizes are indicated on the right with predicted sequence.

### Figure 9

Illustration of the sequencing method without gel electrophoresis, as described in Example 3. The sequencing steps were carried out using the indicated 32P-labelled nucleotides in the order of expected incorporation. The primer was unlabelled. Radioactivity accumulation in each well was recorded after each cycle and is shown on the graphic (left scale) with the predicted number of added bases (right scale).

### Figure 10

Illustration of the sequencing method with fluorescence-labelled nucleotides, as described in Example 4. The template was immobilised in a microplate and the primer was unlabelled. The nucleotides were used in the order ofT, C, T, C, T, C, T, which corresponds to the expected sequence. The fluorescence increase after each step was recorded (arbitrary units) and is shown on the graph with the expected number of added bases. The first step corresponds to non specific signal.

### Figure 11

Illustration of parallel sequencing with fluorescently-labelled nucleotides, as described in Example 5. The template was immobilised on a microplate and different unlabelled primers were used on two locations. The nucleotides were used in the arbitrary order of T, C, T, C on both locations. The fluorescence increase after each step was recorded for each location and is shown on the graphic. The predicted number of bases added to each primer is shown in Table 11.

### EXAMPLES

### Example 1

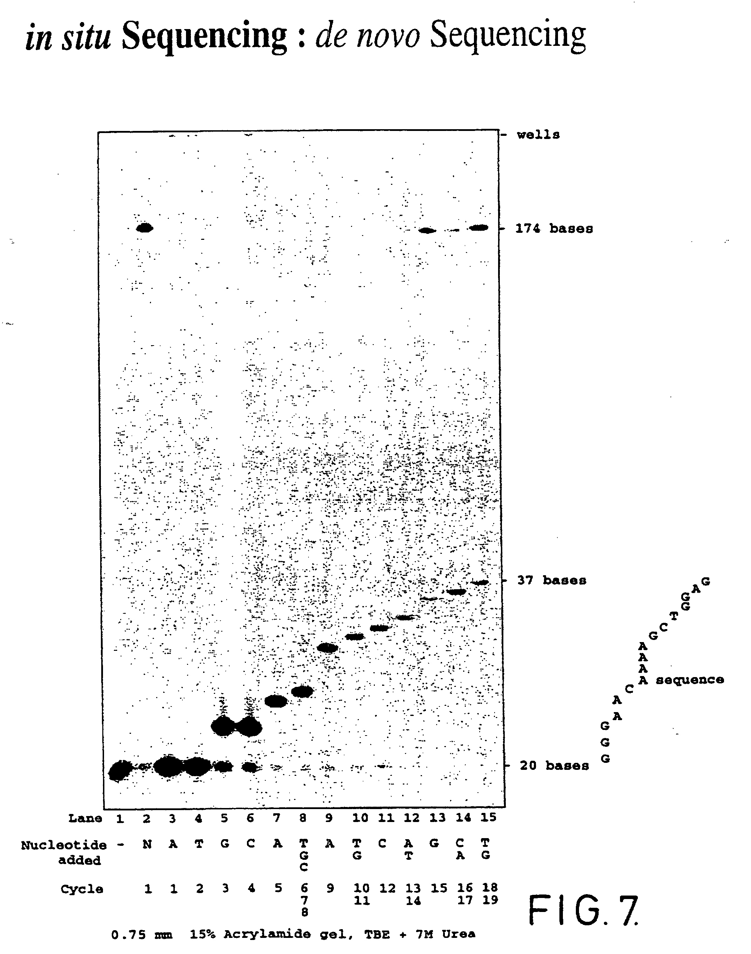

This example provides an indication that successful step-by-step incorporation of nucleotides using the method of the present invention can be achieved. The example uses a sequencing gel for illustrative purposes (see Figure 7) since such gels are routinely used for sequencing. It will however be appreciated that the present invention is preferably used for *in situ* sequencing of nucleic acid molecules and therefore it does not require the use of sequencing gels.

### Experimental procedure:

1. The sequencing cycles were done on single-stranded template molecules bound on magnetic beads.
   The 174 base pair DNA fragment was amplified by PCR from the multiple cloning site of plasmid pBlueScript SK- (Stratagene). The forward primer (5'-biotin-GCGCGTAATACGACTCACTA-3') and reverse primer (5'-CGCAATTAACCCTCACTAAA-3') were located on position 621 and 794, respectively. The amplified double-stranded DNA was bound on streptavidin coated magnetic beads (Dynabeads M-280, Dynal, Oslo) and denatured with NaOH. After washing, the single-stranded DNA bound to the beads was kept in 10 mM tris pH 8.0.
2. The reverse primer was used for sequencing. It was labelled on its 5' end with radioactive phosphate, and annealed to the single-stranded DNA template. The primer molecule is expected to be extended with the following bases : GGGAACAAAAGCTGGAG...
   The primer end-labelling was done with polynucleotide kinase (Pharmacia) in presence of [γ-32P] ATP as described by manufacturer. After purification on Sephadex G-25 spin column, the primer were conserved at -20°C.
   The annealing was performed by heating the mixture of primer and template molecules in 1x Sequenase buffer [40 mM Tris pH 7.5, 20 mM MgCl2, 50 mM NaCl] for 5 min at 70°C and slow cooling for 2 hours.
3. The sequencing reaction were performed by successive cycles of primer extension (in presence of only one type of nucleoside triphosphate), aliquot removal for analysis and washing. Each cycle was done using a different deoxyribonucleoside triphosphate in solution, using the arbitrary order of dATP, dTTP, dGTP, dCTP, dATP, dTTP, dGTP, dCTP, dATP, dTTP, etc.
   The reaction mix contained 0.1 u/µl Sequenase (T7 Sequenase II, Amersham) and 100 nM of a single type of normal deoxyribonucleoside triphosphate in 1x Sequenase buffer. Four different mixes were prepared, each with a different type of nucleoside triphosphate.
   A cycle started with the removal of buffer from the template-bound beads using a magnet. Ice-cold reaction mixture was added. After 5 seconds an aliquot was collected and kept on ice in 10 volumes of stop buffer containing 90% formamide and 20 mM EDTA. Ten volumes of washing buffer (10 mM Tris pH 8.0) were added to the remaining reaction mixture and immediately removed after magnetic separation of the template-bound beads. Washing was repeated and next cycle was initiated.
   At each cycle, a smaller volume of fresh reaction mixture was used, according to the remaining amount of template after aliquot removal and washing losses.
4. The aliquots collected after each cycle were then analysed on denaturing sequencing gel. After migration, the gel was autoradiographed to reveal to position of each band.
   The aliquots in stop buffer were denatured for 5min at 95°C and immediately cooled on ice before loading on 0.75 x 180 x 320 mm 15% acrylamide gels (SE420, Hoefer Scientific Instruments) containing 1xTBE buffer and 7 M urea. Migration was at 2000V for 5min and 250V overnight.
   As a control, unreacted oligonucleotides were applied on gel (lane 1), as well as the product of a reaction performed in presence of all 4 deoxynucleoside triphosphates, to give rise to fully extended molecules (lane 2).
   After migration, the gel was fixed in 10% glacial acetic acid and 10% methanol for 30 min. Still humid, it was transferred in a plastic bag in autoradiography cassette to expose a Kodak X-OMAT AR film for 9h at 25°C.

The very high sensitivity of radio-active label, associated with gel analysis of extended fragments after each cycle allowed careful monitoring of the reaction. As shown on Figure 7 it was demonstrated that:
- Conditions were identified which permit correct polymerisation in the presence of only one type of deoxynucleoside triphosphate. The polymerisation proceeds completely to the last base to be incorporated (lane 5), but is blocked for (further) extension if the complementary base is not present on template molecule (lane 3, 4, or 5).
- There is no misincorporation, as seen on lanes 3, 4, or 6.
- There is no 3'-5' exonuclease activity (e.g. fragments of shorter size are not detected on lane 3 or 4).
- Successive cycles can be carried out correctly.
   Only fragments of the correct size are observed. There are three exceptions : (1) a small proportion of the primer molecules were not extended during first cycle, probably because of 3' end damage of the oligonucleotide. (2) A proportion of full length molecules are observed after cycle 13, which suggests that washing was not complete and that the 4 deoxynucleoside triphosphates were present in solution. This problem is not considered as relevant, because it was not observed between cycles 4 and 12, even though the 4 different deoxynucleosides triphosphates had already been used for reactions. The washing steps should also be facilitated when the experiment will be repeated with template molecules bound on plastic surface. (3) Faint bands of smaller size than expected could be detected after longer exposure, which suggests uncompleted reaction. However, the proportion is so low that it should not interfere with *in situ* detection.
   Only 19 cycles were carried out in this preliminary experiment, but there is no reason why more cycles (up to 50 or up to 100 or more) could not be performed successfully.

This experiment demonstrated that not only re-sequencing (when only the expected deoxynucleoside triphosphate is used for polymerisation), but that also *de novo* sequencing can be carried out efficiently with this method.

After 20 cycles of *de novo* sequencing of an unknown template molecule, the minimal number of bases which could be sequenced is of 6, the maximal number depending on the base order and on the presence of runs of the same base. In this *de novo* sequencing experiment, as many as 17 bases were read in 19 cycles.

It should also be mentioned that the template molecule used here is considered as difficult to sequence because of its high content of palindromic regions, which gives rise to secondary structures in single-stranded DNA. This potential difficulty appeared not to be a problem in our experiment.

### Example 2: Re-sequencing using non-labelled primers

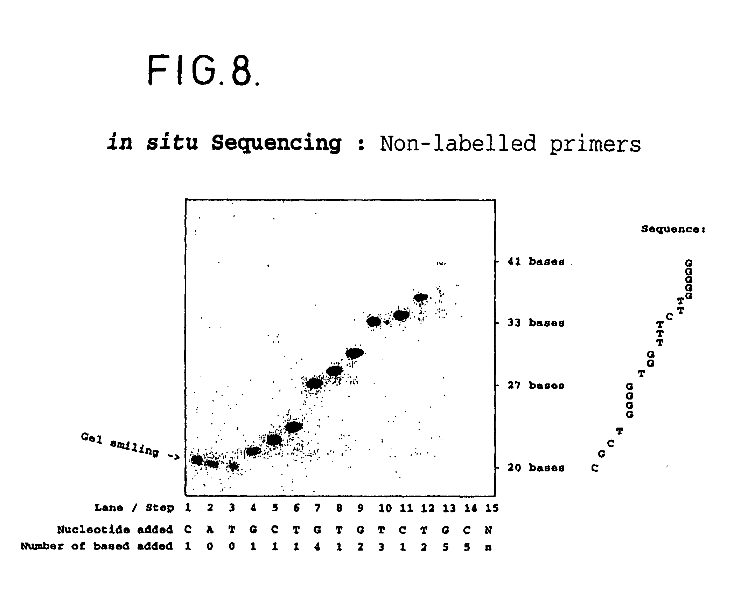

This example provides a further indication that successful step-by-step incorporation of labelled deoxynucleoside triphosphates can be achieved using the method of the invention. Like in Example 1, a sequencing gel was used for illustrative purposes (Figure 8). It will however be appreciated that the present invention is preferably used for *in situ* sequencing of nucleic acid molecules and therefore it does not require the use of sequencing gels.

### Template preparation:

A 608 base pair DNA fragment inserted into the polylinker of pBlueScript SK-plasmid was amplified by PCR. The forward primer (5'-GCG CGT AAT ACG ACT CAC TA-3') was biotinylated on its 5'-end and the reverse primer (5'-GCA ATT AAC CCT CAC TAA A-3') was not functionalised (i.e. -Off). The amplified double-stranded DNA was purified on a spin column (Pharmacia MicroSpin S-400 HR) to remove unused primers. A hundred microlitres of purified DNA were then diluted in a total volume of 400 µl and bound on same volume of streptavidin coated magnetic beads (10 mg/ml) according to the manufacturer's protocol (Dynabeads M-280, Dynal, Oslo). The DNA was denatured in presence of 0.1 N NaOH and beads were washed to remove the complementary strand. The single-stranded DNA bound to the beads was resuspended in 100 µl 10 mM tris pH 8.0 and conserved at 4°C.

### Primer annealing:

The primer 5'-TAC CAG TTT CCA TTC CAG C-3' was used for sequencing. The annealing was performed by heating the primer and template mixture for 5 min at 95°C and cooling to 60°C in 30 min. Typically, 20µl of beads were annealed with 10 pmol of primer in 100µl of 1x Sequenase buffer (40 mM Tris pH 7.5, 20 mM MgCl2, 50 mM NaCl). The primer was expected to be extended with the following bases: CGCTGGGGTGGTTT... which are complementary to the template sequence.

The primer extension reactions were performed in presence of only one type of deoxynucleoside triphosphate. Each step was done using a different deoxyribonucleoside triphosphate in solution, using me expected order of dCTP, dATP, dTTP, dGTP, dCTP, dTTP, dGTP, dTTP, dGTP, dTTP, etc..(the first dATP and dTTP will not get incorporated and serve to demonstrate absence of misincorporation). The T7 DNA polymerase Sequenase 2.0 (Amersham) was chosen for polymerisation reactions. The reaction mix contained 0.2 u/µl Sequenase, 5mM DTT, 250 nM of a single type of normal deoxyribonucleotide and 5 nM of the same type of [α-32P]-labelled deoxyribonucleotide (50 nCi/µl, Amersham) in 1x Sequenase buffer. Four different mixes were prepared, each with a different type of deoxynucleoside triphosphate.

The first polymerisation step was initiated by sedimenting 20 µl of template-bound beads. The beads were resuspended in 25 µl of ice-cold reaction mix and reaction was allowed to proceed for 10 seconds.

The beads were immediately sedimented with the magnet and washed 3 times for 1 min in 10 volumes of 1x Sequenase buffer. After the final wash, the beads were resuspended in the initial volume of 1x Sequenase buffer. One microlitre aliquot was collected after each cycles and kept in 10 µl stop buffer (90% formamide, 1x TBE buffer, 20 mM EDTA, 0.1% Bromophenol Blue, 0.1 % Xylene Cyanol FF).

The next polymerisation and washing steps were performed as described *supra* using adequate volumes of reagents (i.e. each step using smaller volumes to compensate for aliquot removal).

After final washing step, sample were analysed by gel electrophoresis. The aliquots in stop buffer were denatured for 5 min at 95°C and immediately cooled on ice before loading on 0.75 x 180 x 320 mm 15% acrylamide gel containing 1x TBE buffer and 7 M urea. The electrophoresis system was model SE400 from Hoefer Scientific Instruments. Migration was at 2000 V for 5 min and 250 V overnight. After migration, the gel was immediately transferred into a plastic bag to expose a Kodak X-OMAT AR film in an autoradiography cassette for 3 to 16h at 25°C.

As discussed *supra* and shown in Figure 8, this example indicates that the sequencing method of the invention can be successfully adapted for stepwise sequencing from non-labelled primers.

### Example 3: Sequencing template bound in a microplate well

### Template preparation:

In this example, DNA molecules of approximately 700 bases were used as template. They were bound on the plastic surface of a modified microtitre plate as described by the manufacturer (NucleoLink™ from Nunc A/S, Roskilde, Denmark).

Two types of DNA templates (named A and B) were bound to the wells, either individually or mixed (50% each).

### Primer annealing:

A 20 base-long oligonucleotide was used as sequencing primer (5'-GGT CAG GCT GGT CTC GAA CT-3') specific for DNA template B. The annealing was performed by heating the microtitre plate coated with template for 4 min at 94°C and slow cooling to 60°C in 30 min, in presence of 100nM primer (in 20µl 5xSSC buffer +0.1% Tween® 20 per well). The primer was expected to be extended with the following bases: CCCTACCTCA... which are complementary to the template sequence.

### Polymerisation step:

The primer extension reactions were performed in presence of only one type of deoxynucleoside triphosphate. Each step was done using a different deoxynucleoside triphosphate in solution, using the expected order of dCTP, dTTP, dATP, dCTP, dTTP, dCTP, dATP. The Klenow fragment of DNA polymerase I (New England Biolabs) was used for polymerisation reactions. The reaction mix contained 0.25 u/µl Klenow exo-, 50 nM of a single type of normal deoxyribonucleoside triphosphate and 20 nM of the same type of [α-32P]-labelled deoxyribonucleoside triphosphate (50 nCi/µl, Amersham) in 1x Polymerase I buffer (10 mM Tris pH 7.5, 5 mM MgCl2, 7.5 mM dithiothreitol). Four different mixes were prepared, each with a different type of deoxynucleoside triphosphate. Typical reactions were performed in 12 µl.

A polymerisation step was initiated with the removal of reagents from the well. The adequate volume of reaction mixture was added and reaction was allowed to proceed for 30 seconds at room temperature.

### Washing step:

The wells were immediately washed 3 times for 1 min in 100 µl of TNT buffer (100 mM Tris pH 7.5, 150 mM NaCl, 0.1% Tween® 20).

### Detection step:

After each washing step, the radioactivity incorporated on each well was measured and recorded using a scintillation counter.

### Sequencing cycle:

In this example, the polymerisation, washing and detection steps were repeated sequentially.

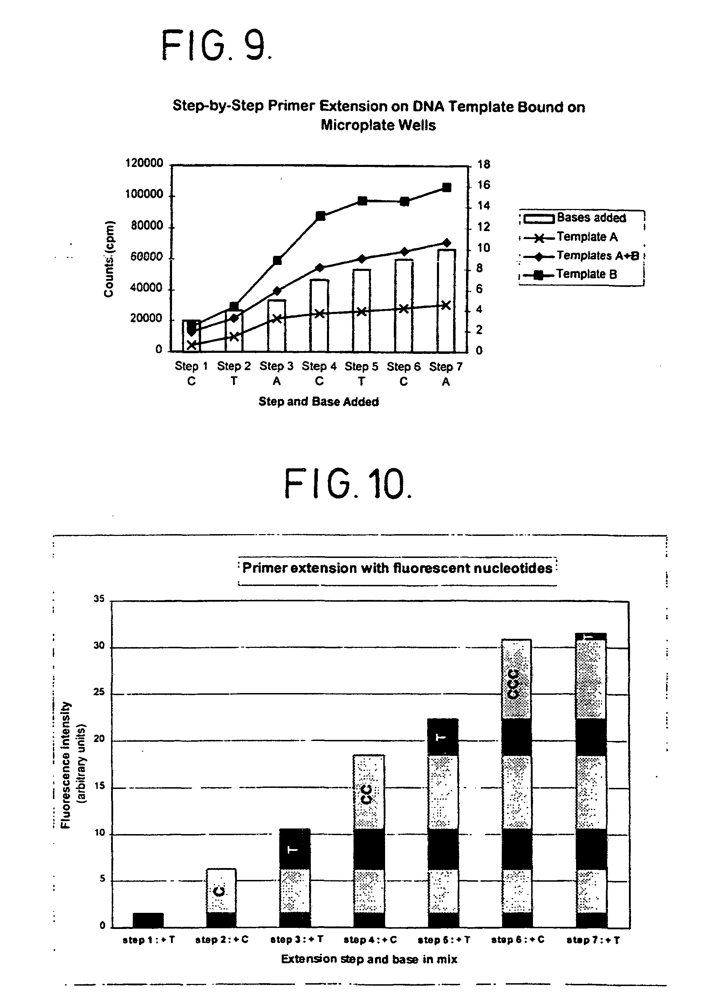

Results shown in Figure 9 indicate that primer extension occurred as expected. The stepwise counts increase occurred specifically in presence of template B, and was dependent on its amount bound on wells (e.g. approximately twice as much counts in presence of 100% template B than in presence of 50% template B). After each step, the number of counts detected increased almost proportionally to the number of bases added.

### Example 4: Sequencing using fluorescent label

This example demonstrates that sequencing can be achieved *in situ* by stepwise incorporation of fluorescently-labelled nucleoside triphosphates. The number of added bases is determined by the measurement of the fluorescence increase after each cycle of addition. Thus DNA sequencing is achieved without requirement of electrophoresis separation method.

### Template preparation:

In this example, a 157 bp DNA fragment was bound to a streptavidin-coated 96-well microtitre plate. The fragment was prepared by PCR amplification with the forward primer being 5'-biotinylated. The reverse primer was not functionalised. The unincorporated primers were removed by purification of the PCR product on a spin column (Pharmacia MicroSpin S-400 HR). After binding to the streptavidin coated microtitre plate wells (LabSystems, Helsinki), the PCR fragments were denatured by NaOH treatment. After washing, only the forward strand remained bound to the wells.

### Primer annealing:

The primer 5'-ACA CGA GCC ACA CTG TCC CAG GGG C-3' was used for sequencing. The annealing was carried out at 25°C with 0.5 µM of primer in 5x SSC buffer.

### Primer extension:

The expected primer extension on the template is : C, T, C, C, T, C, T. The reaction mix [1x Sequenase buffer, 0.15 u/µl T7 DNA polymerase (Sequenase 2.0, Amersham), 0.1 % BSA, 5 mM DTT] contained 0.5 µM of dUTP or dCTP [as a 60% mix of Cy5-labelled (Amersham) and non-labelled deoxynucleotide triphosphates]. The reactions were carried out for 1 min at 25°C in presence of either dUTP or dCTP. After each step, the wells were washed twice with 1x Sequenase buffer and the fluorescence intensity in each well was measured with a cooled CCD camera (Princeton Instruments) mounted on a fluorescence microscope (Zeiss Axiovert TV 100). Note that dTTP can be substituted by dUTP, the two nucleotides having identical specificity.

Results shown on Figure 10 demonstrate that after each cycle of dNTP addition, the measured increase in fluorescence is proportional to the number of bases incorporated. The successive reactions were performed alternatively in presence of dUTP or dCTP. dUTP was used for the first step, which served as a control of non-specific extension.

In this example, 8 bases were sequenced using the method of the invention.

### Example 5: Parallel sequencing using fluorescently-labelled dNTPs

This example demonstrates that *in situ* sequencing by stepwise incorporation of fluorescently-labelled deoxynucleoside-triphosphates can be achieved in parallel in different locations. Thus several samples can be sequenced simultaneously.

### Template preparation:

In this example, a 131 bp DNA fragment was bound to a streptavidin-coated 96-well micro-titre plate. The fragment was prepared by PCR amplification with the forward primer being 5'-biotinylated. The reverse primer was not functionalised. The unincorporated primers were removed by purification of the PCR product on a spin column (Pharmacia MicroSpin S-400 HR). After binding to the streptavidin coated microtitre plate wells (LabSystems, Helsinki), the PCR fragments were denatured by NaOH treatment. After washing, only the forward strand remained bound to the wells.

### Primer annealing:

Two primers were annealed to the template on different locations. Primer 79 (5'-GGG GTT TCT CCA TGT TGG TCA GGC TGG TC-3') was annealed at a first location on the microtitre plate and primer 94 (5'-TGG TCA GGC TGG TCT CGA ACT CCC TAC-3') at a second location along the microtitre plate. The annealing was carried out at 25°C with 0.5 µM of primer in 5x SSC buffer.

### Primer extension:

The expected extension for primer 79 is-: TCG... and for primer 94-: CTCA... The reaction mix [1x Sequenase buffer, 0.15 u/µl T7 DNA polymerase (Sequenase 2.0, Amersham), 0.1 % BSA, 5 mM DTT] contained 0.5 µM of dUTP or dCTP [as a 60% mix of Cy5-labelled (Amersham) and non-labelled deoxynucleotides]. The reactions were carried out for 1 min. at 25°C in presence of either dUTP or dCTP. After each step, the wells were washed twice with 1x Sequenase buffer and the fluorescence intensity in each well was measured with a cooled CCD camera mounted on a fluorescence microscope, as described above.

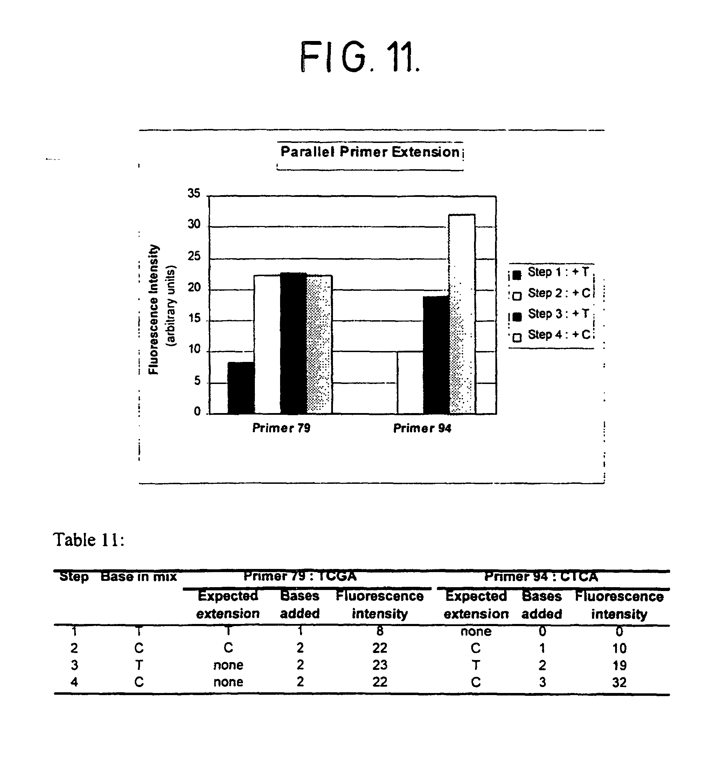

Results shown on Figure 11 demonstrate that after each step and for each primer the fluorescence increase is proportional to the number of bases incorporated. The successive reactions were performed alternatively in presence of DUTP or dCTP. Fluorescence increased on steps 1 and 2 for primer 79, as one base was added on these steps (see table). For primer 94 however, bases were only incorporated on steps 2, 3, and 4. As expected, no increase of fluorescence was detected for primer 79 on steps 3 and 4, and for primer 94 on step 1.

The example shows the specificity of the method and its use for sequencing in parallel simultaneously on different locations.

### Use of double stranded, nicked molecules

Although the foregoing examples have been generally based upon a method in which primers are hybridised to a single-stranded nucleic acid molecule and the primers are then extended, it is possible to use an alternative approach in which a double stranded molecule having a nick is provided (a nick being a gap in one strand of a double-stranded DNA molecule and allowing a free 3'-OH end to be provided).

The nick provides a 3'-OH group which acts as a starting point for chain extension in the presence of a suitable polymerase and a supply of nucleotides. The 3'-OH group provided via a nick therefore has an equivalent function to the 3'-OH group at the end of a primer in the methods discussed previously. The nick can be provided by any suitable means. For example it can be provided by using restriction enzymes on DNA molecules with hemiphosphorothioated recognition sites. (Reference : Spears, P.A., Linn, C.P., Woodard, D.L., Walker, G.T. (1997). Simultaneous strand displacement amplification and fluorescence polarization detection of Chlamidia trachomatis DNA. Analytical Biochemistry 247: 130-137).

## Claims

1. A method for sequencing nucleic acid molecules, comprising the steps of:
a) providing at a first location of a surface a plurality of single stranded nucleic acid molecules that have the same sequences as one another and that are hybridised to primers in a manner to allow primer extension in the presence of nucleotides and a nucleic add polymerase;
b) providing at a second location of the surface, which is different from the first location, a plurality of single stranded nucleic acid molecules that have the same sequences as one another, but that have different sequences from the sequences of the single stranded nucleic acid molecules at the first location, and that are also hybridised to primers in a manner to allow primer extension in the presence of nucleotides and a nucleic add polymerase;
c) providing each location with a nucleic acid polymerase and a given labeled nucleotide under conditions that allow extension of the primers if a complementary base or if a plurality of such bases is present at the appropriate position in the single stranded nucleic add molecules;
d) detecting whether or not said labeled nucleotide has been used for primer extension at each location by determining whether or not the label present on said nudeotide has been incorporated into extended primers;
e) repeating steps c) and d) one or more times so that extended primers comprising a plurality of labels are provided.

2. A method according to claim 1, wherein all or part of the sequence that is obtained in step e) is converted to provide a complementary sequence thereto.

3. A method according to any preceding claim, wherein if the given nucleotide has been used in primer extension in step d) then this step includes the step of detecting how many of the given nucleotides have been used per extended primer by measuring the cumulative increase of the signal generated by the label on said nucleotides.

4. A method according to any preceding claim, wherein after step c) excess nucleotides that have not been used in primer extension are removed (e.g. by washing).

5. A method according to any preceding claim, wherein step d) uses absorption or emission spectrometry, or a Charge-Coupled-Device camera coupled to a magnifying device.

6. A method according to any preceding claim, wherein said single stranded nucleic acid molecules, said primers or both of the aforesaid are immobilised.

7. A method according to any preceding claim that is used to fully or partially sequence 10 or more nucleic acid molecules having different sequences simultaneously.

8. A method according to any preceding claim that is used to fully or partially sequence 100 or more nudeic add molecules having different sequences simultaneously.

9. A method according to any preceding claim that is used to fully or partially sequence 1000 or more nucleic acid molecules having different sequences simultaneously.

10. A method according to any preceding claim, wherein each of four different nucleotides is used in primer extension.

11. A method according to claim 10, wherein said four different nucleotides are used in a predetermined order in repeated cycles.

12. A method according to claim 10 or claim 11, wherein the nucleotides are dATP, dTTP, dGTP and dCTP in labelled form.

13. A method according to claim 10 or claim 11, wherein the nucleotides are ATP, UTP, GTP and CTP in labelled form.

14. A method according to any preceding claim, wherein the detection step is carried out without moving the nucleic acid molecules from the different locations.

15. A method as described in any preceding claim with the exception that double stranded nucleic acid molecules having nicks therein are provided at the first and/or second locations instead of providing single stranded molecules hybridised to primers.

16. A method as described in any preceding daim wherein the given nucleotide in step c) is provided in a mixture containing said nucleotide in labeled and non-labeled form.

17. An apparatus for performing a method according to any preceding claim, the apparatus comprising:
a) nucleic acids arrayed in the form of distinct areas, providing at a first location of a surface a plurality of single stranded nudeic acid molecules that have the same sequences as one another and that are hybridised to primers in a manner to allow primer extension in the presence of nucleotides and a nucleic acid polymerase, and at a second location of the surface, which is different from the first location, a plurality of single stranded nucleic acid molecules that have the same sequences as one another, but that have different sequehces from the sequences of the single stranded nucleic acid molecules at the first location, and that are also hybridised to primers in a manner to allow primer extension in the presence of nucleotides and a nucleic acid polymerase;
b) means for dispensing the sequencing reagents;
c) means for removing the sequencing reagents;
d) automated means to allow repeated cycles of primer extension;
e) detection means for performing step d) of claim 1 or an equivalent step for any of claims 2 to 15, the detection means being adapted to distinguish between said different locations whether or not the label present on the nucleotides has been incorporated into extended primers.

18. The apparatus of claim 17 wherein:
a) the nucleic acids are arrayed in distinct areas on a planar surface;
b) means for dispensing the sequencing reagents are formed by one or more inlets for reagents connected to electronically controlled valves and a reservoir for reagents;
c) means for removing the sequencing regents are formed by an outlet for reagents connected to electronically controlled valves and a reservoir of reagents;
d) automated means for allowing repeated cycles of primer extension and detection are formed by electronically controlled valves for dispensing and removing the reagents;
e) detection means for distinguishing amongst said distinct areas whether or not the label present on the nucleotides has been incorporated into extended primers, are formed by a Charge-Coupled-Device camera coupled to a magnifying device.

## Patentansprüche

1. Ein Verfahren zum Sequenzieren eines Nukleinsäuremoleküls, umfassend die Schritte von:
a) Bereitstellen einer Vielzahl von einzelsträngigen Nukleinsäuremolekülen an einer ersten Stelle einer Oberfläche, wobei die Nukleinsäuremoleküle untereinander die selben Sequenzen aufweisen und in einer Weise an Primer hybridisiert sind, dass Extension der Primer in der Gegenwart von Nukleotiden und einer Nukleinsäure Polymerase ermöglicht ist;
b) Bereitstellen einer Vielzahl von einzelsträngigen Nukleinsäuremolekülen an einer zweiten Stelle der Oberfläche, wobei die Nukleinsäuremoleküle verschieden von der ersten Stelle ist, welche untereinander die selben Sequenzen aufweisen, aber verschiedene Sequenzen im Vergleich zu den Sequenzen der einzelsträngigen Nukleinsäuremoleküle der ersten Stelle aufweisen, und ebenso in einer Weise an Primer hybridisiert sind, dass Extension der Primer in der Gegenwart von Nukleotiden und einer Nukleinsäure Polymerase ermöglicht ist;
c) Versorgen einer jeden Stelle mit einer Nukleinsäure Polymerase und einem gegebenen gelabelten Nukleotid unter Bedingungen, welche die Extension der Primer erlauben, wenn eine komplementäre Base oder eine Vielzahl solcher Basen an der geeigneten Stelle in dem einzelsträngigen Nukleinsäuremolekül vorhanden ist;
d) Detektieren, ob besagte gelabelte Nukleotide für die Extension der Primer an jeder der Stellen verwendet wurden oder nicht, durch Bestimmen, ob der Label auf besagtem Nukleotid in den verlängerten Primer inkorporiert worden ist oder nicht;
e) Wiederholen der Schritte c) und d) einmal oder mehrmals, so dass verlängerte Primer, welche eine Vielzahl von Labeln umfassen, bereitgestellt werden.

2. Das Verfahren gemäß Anspruch 1, worin die gesamte oder Teile der Sequenz, welche in Schritt e) erhalten wird, konvertiert wird, um eine komplementäre Sequenz davon bereitzustellen.

3. Ein Verfahren gemäß irgendeinem der vorangehenden Ansprüche, worin, falls das gegebene Nukleotid für die Extension der Primer in Schritt d) verwendet wurde, dann dieser Schritt den Schritt des Detektierens, wie viele der gegebenen Nukleotide pro verlängerten Primer verwendet wurden, durch Messung der kumulativen Vergrößerung des Signals, welches durch die Label auf den besagten Nukleotiden generiert wird, einschließt.

4. Ein Verfahren gemäß irgendeinem der vorangehenden Ansprüche, worin nach Schritt c) überschüssige Nukleotide, welche nicht für die Extension der Primer verwendet wurden, entfernt werden (zB. durch Waschen).

5. Ein Verfahren gemäß irgendeinem der vorangehenden Ansprüche, worin Schritt d) Absorptions- oder Emissions-Spektroskopie nutzt, oder eine Charged-Coupled-Device Kamera gekoppelt an eine Verstärkereinheit.

6. Ein Verfahren gemäß irgendeinem der vorangehenden Ansprüche, worin besagte einzelsträngige Nukleinsäuremoleküle, besagte Primer oder beide der vorhergenannten immobilisiert sind.

7. Ein Verfahren gemäß irgendeinem der vorangehenden Ansprüche, welches genutzt wird, um vollständig oder teilweise 10 oder mehr Nukleinsäuremoleküle, welche verschiedene Sequenzen aufweisen, gleichzeitig zu sequenzieren.

8. Ein Verfahren gemäß irgendeinem der vorangehenden Ansprüche, welches genutzt wird, um vollständig oder teilweise 100 oder mehr Nukleinsäuremoleküle, welche verschiedene Sequenzen aufweisen, gleichzeitig zu sequenzieren.

9. Ein Verfahren gemäß irgendeinem der vorangehenden Ansprüche, welches genutzt wird, um vollständig oder teilweise 1000 oder mehr Nukleinsäuremoleküle, welche verschiedene Sequenzen aufweisen, gleichzeitig zu sequenzieren.

10. Ein Verfahren gemäß irgendeinem der vorangehenden Ansprüche, worin jedes der vier verschiedenen Nukleotide bei der Extension der Primer verwendet wird.

11. Ein Verfahren gemäß Anspruch 10, worin besagte vier verschiedenen Nukleotide in einem prädeterminierten oder in wiederholten Zyklen verwendet werden.

12. Ein Verfahren gemäß Anspruch 10 oder Anspruch 11, worin die Nukleotide dATP, dTTP, dGTP und dCTP in gelabelter Form sind.

13. Ein Verfahren gemäß Anspruch 10 oder Anspruch 11, worin die Nukleotide ATP, TTP, GTP und CTP in gelabelter Form sind.

14. Ein Verfahren gemäß irgendeinem der vorangehenden Ansprüche, worin der Detektionsschritt ohne Bewegen der Nukleinsäure Moleküle von den verschiedenen Stellen durchgeführt wird.

15. Ein Verfahren wie in irgendeinem der vorangehenden Ansprüche beschrieben mit der Ausnahme, dass doppelsträngige Nukleinsäuremoleküle mit einem Nick darin an der ersten und / oder zweiten Stelle bereitgestellt werden an Stelle der Bereitstellung von einzelsträngigen Molekülen, welche hybridisiert an Primer sind.

16. Ein Verfahren wie in irgendeinem der vorangehenden Ansprüche beschrieben, worin das gegebene Nukleotid in Schritt c) in einer Mischung enthaltend besagte Nukleotide in gelabelter und ungelabelter Form bereitgestellt wird.

17. Ein Apparat zum Durchführen eines Verfahrens gemäß irgendeinem der vorangehenden Ansprüche, wobei der Apparat folgendes umfasst:
a) Nukleinsäuren angeordnet in der Form von abgetrennten Arealen, wobei an einer ersten Stelle einer Oberfläche eine Vielzahl von einzelsträngigen Nukleinsäuremoleküten, welche untereinander die selben Sequenzen aufweisen und welche an Primer hybridisiert sind, in einer Weise, dass Extension der Primer in der Gegenwart von Nukleotiden und einer Nukleinsäure Polymerase ermöglicht ist; bereitgestellt wird und eine Vielzahl von einzelsträngigen Nukleinsäuremolekülen an einer zweiten Stelle der Oberfläche, welche verschieden von der ersten Stelle ist, welche untereinander die selben Sequenzen aufweisen, aber verschiedene Sequenzen im Vergleich zu den Sequenzen der einzelsträngigen Nukleinsäuremoleküle der ersten Stelle aufweisen, und welche ebenso an Primer hybridisiert sind in einer Weise, dass Extension der Primer in der Gegenwart von Nukleotiden und einer Nukleinsäure Polymerase ermöglicht ist, bereitgestellt wird;
b) Mittel zur Verabreichung der sequenzierenden Agenzien;
c) Mittel zur Beseitigung der sequenzierenden Agenzien;
d) Automatisierte Mittel, um wiederholte Zyklen der Extension der Primer zu ermöglichen,
e) Mittel zur Detektion zur Durchführung von Schritt d) von Anspruch 1 oder einem äquivalenten Schritt für irgendeinen der Ansprüche 2 bis 15, wobei die Detektionsmittel angepasst sind, um zwischen den verschiedenen Stellen zu unterscheiden, ob der Label, welcher auf den Nukleotiden vorhanden ist, in die verlängerten Primer inkorporiert wurde oder nicht.

18. Der Apparat gemäß Anspruch 17, worin:
a) Die Nukleinsäuren in verschiedenen Arealen auf einer planaren Oberfläche angeordnet sind;
b) Mittel zur Verabreichung der sequenzierenden Agenzien durch einen oder mehrere Einlassvorrichtungen für Reagenzien gebildet werden, welche mit elektronisch kontrollierten Ventile verbunden sind und mit einem Reservoir für Reagenzien;
c) Mittel zur Entfernung der sequenzierenden Agenzien durch eine Auslassvorrichtung gebildet werden, welche mit elektronisch kontrollierten Ventile verbunden ist und mit einem Reservoir der Reagenzien;
d) Automatisierte Mittel, um wiederholte Zyklen der Extension und der Detektion der Primer zu ermöglichen, durch elektronisch kontrollierte Ventile zur Verabreichung und Entfernung der Reagenzien gebildet werden;
e) Mittel zur Detektion für die Unterscheidung zwischen besagten abgetrennten Arealen, ob das Label, welches auf den Nukleotiden vorhanden ist, in die verlängerten Primer inkorporiert wurde oder nicht, durch eine Charged-Coupled-Device Kamera gekoppelt an einen Verstärker gebildet werden.

## Revendications

1. Méthode de séquençage de molécules d'acide nucléique, comprenant les étapes consistant à :
a) fournir en un premier emplacement d'une surface une pluralité de molécules d'acide nucléique simple brin qui ont les mêmes séquences les unes que les autres et qui sont hybridées à des amorces de manière à permettre l'extension d'amorce en présence de nucléotides et d'une polymérase d'acide nucléique ;
b) fournir en un second emplacement de la surface, qui soit différent du premier emplacement, une pluralité de molécules d'acide nucléique simple brin qui ont les mêmes séquences les unes que les autres, mais qui ont des séquences différentes des séquences des molécules d'acide nucléique simple brin sur le premier emplacement et qui sont aussi hybridées à des amorces de manière à permettre l'extension d'amorce en présence de nucléotides et d'une polymérase d'acide nucléique ;
c) fournir à chaque emplacement une polymérase d'acide nucléique et un nucléotide marqué donné dans des conditions qui permettent l'extension d'amorce si une base complémentaire ou si une pluralité de telles bases est présente à l'emplacement approprié dans les molécules d'acide nucléique simple brin ;
d) détecter si ledit nucléotide marqué a été ou non utilisé pour l'extension d'amorce à chaque emplacement en déterminant si le marqueur présent sur ledit nucléotide a ou non été incorporé aux amorces étendues ;
e) répéter les étapes c) et d) une ou plusieurs fois de sorte que des amorces étendues comprenant une pluralité de marqueurs soient fournies.

2. Méthode selon la revendication 1, dans laquelle tout ou partie de la séquence qui est obtenue à l'étape e) est convertie pour fournir sa séquence complémentaire.

3. Méthode selon l'une quelconque des revendications précédentes, dans laquelle le nucléotide donné a été utilisé dans l'extension d'amorce à l'étape d) puis cette étape inclut l'étape consistant à détecter combien des nucléotides donnés ont été utilisés par amorce étendue en mesurant l'augmentation cumulée du signal généré par le marqueur sur lesdits nucléotides.

4. Méthode selon l'une quelconque des revendications précédentes, dans laquelle après l'étape c) les nucléotides en excès qui n'ont pas été utilisés dans l'extension d'amorce sont éliminés (par exemple par lavage).

5. Méthode selon l'une quelconque des revendications précédentes, dans laquelle l'étape d) utilise l'absorption ou la spectrométrie d'émission ou un dispositif de caméra à charge couplée, couplé à un dispositif de loupe.

6. Méthode selon l'une quelconque des revendications précédentes, dans laquelle lesdites molécules d'acide nucléique simple brin, lesdites amorces ou les deux sont immobilisées.

7. Méthode selon l'une quelconque des revendications précédentes qui est utilisée pour séquencer complètement ou partiellement 10 molécules d'acide nucléique ou plus ayant des séquences différentes simultanément.

8. Méthode selon l'une quelconque des revendications précédentes qui est utilisée pour séquencer complètement ou partiellement 100 molécules d'acide nucléique ou plus ayant des séquences différentes simultanément.

9. Méthode selon l'une quelconque des revendications précédentes qui est utilisée pour séquencer complètement ou partiellement 1 000 molécules d'acide nucléique ou plus ayant des séquences différentes simultanément.

10. Méthode selon l'une quelconque des revendications précédentes, dans laquelle chacun de quatre nucléotides différents sont utilisés dans l'extension d'amorce.

11. Méthode selon la revendication 10, dans laquelle lesdits quatre nucléotides différents sont utilisés dans un ordre prédéterminé au cours de cycles répétés.

12. Méthode selon la revendication 10 ou selon la revendication 11, dans laquelle les nucléotides sont dATP, dTTP, dGTP et dCTP sous forme marquée.

13. Méthode selon la revendication 10 ou la revendication 11, dans laquelle les nucléotides sont ATP, UTP, GTP et CTP sous forme marquée.

14. Méthode selon l'une quelconque des revendications précédentes, dans laquelle l'étape de détection est réalisée sans déplacer les molécules d'acide nucléique des différents emplacements.

15. Méthode comme décrite dans l'une quelconque des revendications précédentes à l'exception que des molécules d'acide nucléique double brin contenant des césures sont fournies aux premier et/ou second emplacements au lieu de fournir des molécules simple brin hybridées à des amorces.

16. Méthode comme décrit dans l'une quelconque des revendications précédentes dans laquelle le nucléotide donné à l'étape c) est fourni dans un mélange contenant ledit nucléotide sous forme marquée et non marquée.

17. Appareil pour mettre en oeuvre une méthode selon l'une quelconque des revendications précédentes, l'appareil comprenant :
a) des acides nucléiques arrangés sous la forme de zones distinctes, fournissant en un premier emplacement d'une surface une pluralité de molécules d'acide nucléique simple brin qui ont les mêmes séquences les unes que les autres et qui sont hybridées à des amorces de manière à permettre l'extension d'amorce en présence de nucléotides et d'une polymérase d'acide nucléique et en un second emplacement de la surface, qui soit différent du premier emplacement, une pluralité de molécules d'acide nucléique simple brin qui ont les mêmes séquences les unes que les autres, mais qui ont des séquences différentes des séquences des molécules d'acide nucléique simple brin sur le premier emplacement et qui sont aussi hybridées à des amorces de manière à permettre l'extension d'amorce en présence de nucléotides et d'une polymérase d'acide nucléique ;
b) des moyens pour délivrer les réactifs de séquençage ;
c) des moyens pour retirer les réactifs de séquençage;
d) des moyens automatisés pour permettre des cycles répétés d'extension d'amorce ;
e) des moyens de détection pour réaliser l'étape d) de la revendication 1 ou une étape équivalente de l'une quelconque des revendications 2 à 15, les moyens de détection étant adaptés pour faire la distinction entre lesdits différents emplacements, que le marqueur présent sur les nucléotides ait été incorporé ou non aux amorces étendues.

18. Appareil selon la revendication 17, dans lequel :
a) les acides nucléiques sont arrangés en zones distinctes sur une surface plane ;
b) les moyens pour délivrer les réactifs de séquençage sont formés par une ou plusieurs entrées pour les réactifs, raccordées à des valves contrôlées électroniquement et à un réservoir pour les réactifs ;
c) les moyens pour retirer les réactifs de séquençage sont formés par une sortie pour les réactifs raccordée à des valves contrôlées électroniquement et à un réservoir de réactifs ;
d) les moyens automatisés pour permettre des cycles répétés d'extension d'amorce et de détection sont formés par des valves contrôlées électroniquement pour délivrer et retirer les réactifs ;
e) les moyens de détection pour distinguer lesdites zones distinctes, que le marqueur présent sur les nucléotides ait été incorporé ou non aux amorces étendues, sont formés par un dispositif de caméra à charge couplée, couplé à une loupe.
